Europäisches Patentamt

European Patent Office

Office européen des brevets

(11) Publication number: **0 310 362 B1**

(12) **EUROPEAN PATENT SPECIFICATION**

(45) Date of publication of patent specification: **30.03.94**    (51) Int. Cl.⁵: **A61K 39/215**, A61K 39/12

(21) Application number: **88309003.7**

(22) Date of filing: **29.09.88**

(54) **Vaccine for protection of cats against feline infectious peritonitis.**

(30) Priority: **01.10.87 US 103144**

(43) Date of publication of application:
**05.04.89 Bulletin  89/14**

(45) Publication of the grant of the patent:
**30.03.94 Bulletin  94/13**

(84) Designated Contracting States:
**AT BE CH DE ES FR GB GR IT LI LU NL SE**

(56) References cited:
**EP-A- 0 011 864**
**EP-A- 0 027 347**
**WO-A-87/04624**
**US-A- 4 195 130**

(73) Proprietor: **NORDEN LABORATORIES, INC.**
**601 W. Cornhusker Highway**
**Lincoln, Nebraska 68501(US)**

(72) Inventor: **Gerber, Jay Dean**
**2435 Stockwell Street**
**Lincoln, NE 68502(US)**
Inventor: **Ingersoll, Jerald Dee**
**5901 Lillybridge No.2**
**Lincoln, NE 68506(US)**

(74) Representative: **West, Vivien et al**
**SmithKline Beecham**
**Corporate Intellectual Property**
**Great Burgh**
**Yew Tree Bottom Road**
**Epsom, Surrey KT18 5XO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 310 362 B1

**Description**

Field of Invention

This invention relates to a method of preparation of a vaccine useful for the immunization of cats against the feline infectious peritonitis (FIP) virus. More specifically it relates to an attenuated, temperature sensitive vaccine which effectively protects cats from FIP.

Background of the Invention

Feline infectious peritonitis (FIP) is a disease of both domestic and wild cats. The virus affects most of the internal organs of the animal and is almost always fatal. The virus is highly contagious, affecting kittens as well as adult cats.

The FIP virus was identified as a coronavirus by Horzinek and Osterhaus, Arch. Virol. 59:1(1979). FIP virus is related to transmissible gastroenteritis virus (TGEV) of pigs, enteric coronavirus of dogs and a respiratory coronavirus of man. There is also a feline enteric coronavirus (FECV) that replicates mainly in the intestine and causes only a mild diarrheal disease. Lutz, et al., J. Small Animal Pract. 27:108(1986).

Although it is known that FIP is caused by a coronavirus, the manner in which the infection is transmitted among cats and its pathogenesis are still poorly understood. The pathogenesis of the disease is very complex and studies indicate that host, viral and environmental factors play a role in the form and progression of the disease. Pedersen, et al., 34th Annual Symposium, Viral Diseases of Small Animals 7:1001(1985).

FIP can occur in two different forms: the wet or effusive form characterized by a fibrinous peritoneal exudate and the dry or parenchymatous form which is characterized by granulomatous inflammation of different organs and little or no exudate. Lutz, et al., supra.

Because there are other coronavirus infections of cats, for example, FECV, which are antigenically related to FIP virus, the pathogenesis of FIP has been difficult to characterize. As a result, serological tests for diagnosis of the disease have lacked specificity and have confused the interpretation of earlier studies. Pederson, Feline Practice 13:13(1983).

Until recently, protective active immunization against FIP was not possible. On the contrary, vaccinated cats were more susceptible to disease. Pedersen, et al., Am. J. Vet. Res. 44:229(1983); Weiss, et al., Comp. Immun. Microbiol. Infect. Dis. 4:175(1981); Weiss, et al., Am. J. Vet. Res. 41:663(1980).

Cats are infected by the oronasal route. FIP virus multiplies in epithelial cells of the upper respiratory tract and intestine. Clinically apparent FIP occurs after the virus crosses the mucosal barrier and causes an immune mediated disease. Lutz, et al., supra., Weiss, et al., Am. J. Vet. Res. 42:382(1981).

Stimulation of a nasal mucosal immune response is best done by intranasal administration of a vaccine. Bienenstock, et al., Immunology 41:249(1980); Murray, The Veterinary Record Nov. 10th:500(1973). Mucosal B-lymphocytes, stimulated to secrete anti-FIP virus IgA antibody, will also migrate to the gut mucosa and also confer local gut immunity. Murray, supra.

WO-A-8 704 624 discloses the use of attenuated FIP-virus as a vaccine albeit without the feature of temperature sensitivity of the FIP-virus.

Summary of Invention

One embodiment of the invention is a FIP vaccine for oral or intranasal administration capable of inducing immunity against infection by FIP virus in felines without serious side effects. Such vaccine comprises an effective amount of an attenuated temperature sensitive (ts) FIP virus.

In another embodiment of the invention, a method of preparation of a vaccine against FIP virus is described which comprises attenuating FIP virus by high passage in susceptible cells; exposing attenuated FIP virus to a mutagenic agent; culturing attenuated FIP virus which after exposure to the mutagenic agent is now temperature sensitive; and collecting the attenuated ts-FIP virus.

In still another embodiment of the invention, a method of immunizing cats against FIP virus is described comprising administering by an oral or intranasal route an attenuated temperature sensitive FIP virus.

In another embodiment of the invention, a FIP virus useful in the production of a vaccine is described. Such FIP virus is attenuated and temperature sensitive.

In another embodiment of the invention, a vaccine dosage unit for inducing immunity to infection by FIP virus is described which comprises 0.5 to 1.5 ml of a liquid, suitable for intranasal or oral administration to a feline, containing $10^2$ to $10^7$ $TCID_{50}$ of an attenuated temperature sensitive FIP virus.

In yet another embodiment of the invention is described a combination vaccine for oral or intranasal administration capable of inducing immunity in felines to infection by FIP virus and one or more other pathogenic organisms or viruses without serious side effects comprising an effective amount of attenuated ts-FIP virus and effective amounts of one or more other antigens protective against infection by another pathogenic organism or virus.

Detailed Description of the Invention

An effective FIP vaccine should stimulate a strong mucosal immune response in order to prevent an infection from crossing the mucosal barrier and a cell-mediated immune (CMI) response that will immediately halt the spread of virus if it crosses the mucosa. The temperature sensitive FIP virus (ts-FIP virus) of the invention may be given intranasally or orally. In the preferred embodiment of the invention the ts-FIP virus is administered intranasally. When it is given intranasally, the ts-FIP virus, because of its ability to grow at temperatures present in the nasopharynx region, readily propagates and stimulates a CMI response and local immune response. In addition, ts-FIP virus stimulates a CMI response to FIP virus following challenge which is not detected in cats vaccinated systemically or in cats infected with virulent FIP virus.

The FIP virus used to prepare the vaccine of this invention is isolated from organs or tissues, preferably the liver, of animals infected with the virus. The organs or tissues are ground up and given orally to the experimental animals, preferably to specific pathogen free (SPF) cats.

After several in vivo passages of infected organs, preferably five in vivo passages of infected spleens or livers, the FIP virus is isolated. The FIP virus is isolated from the infected organs using standard procedures known in the art and cocultured with feline cells. The FIP virus grows readily with feline cells from any source, for example, spleen, mesenteric lymph node, endothelial cells or embryonic cell cultures such as taught by Davis, U.S. Patent 4,303,644 and Fishman, et al., U.S. Patent 4,571,386. In the preferred embodiment of the invention, the FIP virus is isolated from the cells of the diseased tissue and cocultured with feline kidney cells.

The virus extracted from the diseased tissues is cocultured with feline cells in order to adapt the virus to in vitro propagation. In vitro propagation is demonstrated by formation of multinucleated, syncytial cells along with other known cytopathic effects ("cpe") typical of coronaviruses. The virus is passaged until attenuation and then is mutated in order to be made temperature sensitive. In the art, attenuated is defined to mean the virus has been modified in such a manner as to be no longer capable of causing disease. Finally, the attenuated, temperature sensitive virus is administered to cats through either an oral or intranasal route.

In a preferred embodiment, the FIP virus is attenuated by high passage, for example, at least 60 passages, preferably 95-100 passages, in feline kidney cells at between 30 and 35°C. For development of a temperature sensitive FIP virus, an aliquot of culture fluid, at a high passage number is exposed to a mutagenic agent, e.g., chemical or irradiation; preferably ultraviolet irradiation for 3 to 5 minutes, until virus titer following propagation at about 31°C is at least $1 \times 10^2$ $TCID_{50}$, preferably about $1 \times 10^5$ $TCID_{50}$, greater than at 39°C. ($TCID_{50}$ is the tissue culture infective dose which produces a cytopathic effect in 50% of the cultured cells exposed to the virus.)

To obtain optimum virus viability, the viral fluids are collected every 5 minutes and stored at -70°C. Ten-fold serial dilutions ($10^0$ to $10^{-7}$) of each 5 minute sample are tested for virus viability on confluent feline kidney cell monolayers. The optimal sample, preferably the 5 minute sample, is selected and propagated at 31°C at various dilutions. Viral fluids from wells containing single plaques are collected.

Table 1 shows the effect of ultraviolet-irradiation of a virulent strain of FIP virus (DF2-FIP virus) at 31°C. FIP virus was completely inactivated after exposure to ultraviolet light for 10 minutes.

3

EP 0 310 362 B1

Table 1

Effect of Time of UV-Irradiation on the Virus Titer

| Sample | Number of Virus Infected Wells/Dilution | | | | | | | | $TCID_{50}$ Titer |
|---|---|---|---|---|---|---|---|---|---|
| | $10^0$ | $10^{-1}$ | $10^{-2}$ | $10^{-3}$ | $10^{-4}$ | $10^{-5}$ | $10^{-6}$ | $10^{-7}$ | |
| Pre-UV | 4/4[a] | 4/4 | 4/4 | 4/4 | 4/4 | 4/4 | 4/4 | 1/4 | $\geq 10^{6.67}$ |
| 5 minute | 4/4 | 3/4 | 0/4 | 0/4 | 0/4 | 0/4 | 0/4 | 0/4 | $10^{1.33}$ |
| 10 minute | 0/4 | 0/4 | 0/4 | 0/4 | | | | | 0 |
| 15 minute | 0/4 | 0/4 | 0/4 | 0/4 | | | | | 0 |

[a]Number of FIPV infected wells/total number of wells.

FIP virus fluids at a 1:16 dilution exposed to ultraviolet-irradiation for 5 minutes contained viral plaques. Single plaques are propagated in a majority of the wells.

Individual single plaques are titrated in ten fold dilutions ($10^{-3}$ to $10^{-8}$) in 24 well plates at 31°, 37° and 39° C (Table 2).

4

Table 2

Titration of UV-Irradiated Viral Plaques at
31°, 37°, and 39°C

| Plaque No. | Temperature °C | Number of Virus-Infected Wells/Dilution | | | | | | TCID$_{50}$ Titer |
|---|---|---|---|---|---|---|---|---|
| | | $10^{-3}$ | $10^{-4}$ | $10^{-5}$ | $10^{-6}$ | $10^{-7}$ | $10^{-8}$ | |
| 1 | 31 | 4/4 | 4/4 | 4/4 | 4/4 | 2/4 | 0/4 | 7.00 |
| | 37 | 4/4 | 4/4 | 4/4 | 4/4 | 0/4 | 0/4 | 6.50 |
| | 39 | 4/4 | 4/4 | 4/4 | 2/4 | 1/4 | 0/4 | 6.23 |
| 2 | 31 | 4/4 | 4/4 | 4/4 | 4/4 | 4/4 | 0/4 | 7.50 |
| | 37 | 4/4 | 4/4 | 4/4 | 4/4 | 3/4 | 0/4 | 7.33 |
| | 39 | 4/4 | 4/4 | 4/4 | 2/4 | 0/4 | 0/4 | 6.00 |
| 3 | 31 | 4/4 | 4/4 | 4/4 | 4/4 | 4/4 | 1/4 | 7.67 |
| | 37 | 4/4 | 4/4 | 4/4 | 4/4 | 1/4 | 1/4 | 6.88 |
| | 39 | 4/4 | 4/4 | 4/4 | 3/4 | 0/4 | 0/4 | 6.33 |
| 4 | 31 | 4/4 | 4/4 | 4/4 | 4/4 | 4/4 | 0/4 | 7.50 |
| | 37 | 4/4 | 4/4 | 4/4 | 3/4 | 1/4 | 0/4 | 6.50 |
| | 39 | 0/4[a] | 0/4[b] | 0/4 | 0/4 | 0/4 | 0/4 | ≤3.50 |

[a]Four wells displayed scarring, or indications of early infection healed over.
[b]Three wells displayed scarring, or indication of early infection healed over.

The optimum conditions for propagating the virus are based on its ability to propagate at permissive temperatures. At 39° C the TCID$_{50}$ is at least about 1 x 10$^2$ units lower than the TCID$_{50}$ at 31° C and is preferably at least about 1 x 10$^5$ units lower.

Table 3

A Comparison of Viral Propagation for 5 in vitro Passages of a
Temperature-Sensitive Plaque-Isolated FIPV at 31°C and 39°C

Number of Virus-Infected Wells/Dilution

| Passage | Temperature | $10^{-1}$ | $10^{-2}$ | $10^{-3}$ | $10^{-4}$ | $10^{-5}$ | $10^{-6}$ | $10^{-7}$ | $10^{-8}$ | $TCID_{50}$ Titer |
|---|---|---|---|---|---|---|---|---|---|---|
| 1 | 31° | 4/4[a] | 4/4 | 4/4 | 4/4 | 4/4 | 3/4 | 0/4 | 0/4 | 6.33 |
|   | 39° | 4/4 | 0/4 | 0/4 | 0/4 | 0/4 | 0/4 | 0/4 | 0/4 | 1.50 |
| 2 | 31° | 4/4 | 4/4 | 4/4 | 4/4 | 4/4 | 1/4 | 0/4 | 0/4 | 5.67 |
|   | 39° | 4/4 | 0/4 | 0/4 | 0/4 | 0/4 | 0/4 | 0/4 | 0/4 | 1.50 |
| 3 | 31° | 4/4 | 4/4 | 4/4 | 4/4 | 3/4 | 1/4 | 0/4 | 0/4 | 5.50 |
|   | 39° | 0/4 | 0/4 | 0/4 | 0/4 | 0/4 | 0/4 | 0/4 | 0/4 | $\leq$1.50 |
| 4 | 31° | 4/4 | 4/4 | 4/4 | 4/4 | 4/4 | 1/4 | 0/4 | 0/4 | 5.67 |
|   | 39° | 0/4 | 0/4 | 0/4 | 0/4 | 0/4 | 0/4 | 0/4 | 0/4 | $\leq$1.50 |
| 5 | 31° | 4/4 | 4/4 | 4/4 | 4/4 | 4/4 | 2/4 | 0/4 | 0/4 | 6.00 |
|   | 39° | 0/4 | 0/4 | 0/4 | 0/4 | 0/4 | 0/4 | 0/4 | 0/4 | $\leq$1.50 |

[a] Number of FIPV infected wells/Total number of wells.

The attenuated temperature sensitive FIP virus of the present invention can be administered to felines to protect the animals from FIP virus. A dosage is selected which is safe, i.e., does not cause serious side effects and is effective, i.e., induces a protective cellular and local immune response following administration.

The FIP vaccine can be administered intranasally or orally. The preferred mode of administration is intranasal, a typical dose being $10^2$ to $10^7$ $TCID_{50}$ in a volume of 0.5 to 1.5 ml.

The dose may be administered as a single dose or as several dilute doses over a period of time. A single dose of $10^{5.5}$ $TCID_{50}$ virus in 0.5 ml of carrier is preferred with one half of 0.5 ml (0.25 ml) administered by, for example, spray or squeeze bottle or by dropper to each nostril of the animal. Experimental results indicate an effective vaccinal dose can be administered as a single dose, two doses being preferred.

The FIP vaccine can be administered directly, i.e., the virus is grown in cell culture fluid and the culture fluid is then used as the carrier for administration. The culture fluid can be diluted or concentrated by standard techniques to achieve the desired concentration of attenuated ts-FIP virus. Alternatively, a pharmaceutically acceptable preparation of ts-FIP virus can be achieved by extracting the attenuated, temperature sensitive virus from the cell culture by, for example, sucrose density centrifugation separation techniques and mixing with a suitable carrier, for example, water, saline, cholera toxoid, or ovalbumin or with an adjuvant, e.g., Quil A, alhydrogel or an oil emulsion.

In the preferred embodiment, the virus is administered in the culture fluid and aliquots of single dose amounts are prepared. The avirulent, ts-FIP virus may also be lyophilized in single dosage amounts and stored until use. When stored as a lyophilized powder, the vaccine is reconstituted prior to administration in water, saline, culture fluid or other appropriate carriers suitable for direct intranasal administration in cats.

Measurements of antibody response are determined using standard methods, such as enzyme linked immunosorbent assay (ELISA) and a serum virus neutralization (VN) test, and are conducted on blood samples from individual animals. Measurements are made optimally on samples following vaccination and challenge. The lymphocyte blastogenesis procedure as described by Pedersen, et al., The Compendium on Continuing Education 7:1001(1985) is used to show a cell-mediated (lymphocyte) response following vaccination and challenge.

A further aspect of this invention is the preparation and use of combination vaccines consisting of vaccinal amounts of the ts-FIP virus and one or more known feline viruses. For example, combination vaccines can be prepared for oral or intranasal administration consisting of the ts-FIP virus component and one or more feline viruses known to be causative of respiratory infections, e.g., calicivirus, feline herpes (rhinotracheitis) virus.

Other combination vaccines capable of inducing immunity in cats to infection by FIP virus and one or more other pathogenic organisms or viruses can also be prepared utilizing the ts-FIP virus component and other non-respiratory related virus components or pathogenic organisms, e.g., feline distemper (panleukopenia), chlamydia, feline leukemia.

Subunit vaccines can also be prepared. The ts-FIP virus can be combined with subunits of killed or attenuated pathotens, for example, subunit components of feline distemper virus, calicivirus, feline herpes virus or the like for parenteral or intranasal administration. The ts-FIP virus may also be combined with feline leukemia virus vaccine for parenteral administration.

The preparation and use of such combination vaccines is carried out according to procedures as described herein or within the knowledge of those skilled in the art of vaccine production and use.

### Examples

The examples which follow are illustrative and are not limiting of the invention.

### Example 1: Efficacy Test 1

Fourteen SPF male cats (26 months of age) from Liberty Labs (Liberty, New Jersey) were used in the first vaccination-challenge test. Vaccinated cats were kept in one isolation room, nonvaccinated cats in another.

Ten cats were vaccinated 3 times intranasally (IN) 3 weeks apart with ts-FIP virus. Five cats were vaccinated with ts-FIP virus with a virus titer of $10^{5.5}$ $TCID_{50}$/ml at 31° C and five with a virus titer of $10^{3.5}TCID_{50}$/ml at 31°C. The ten vaccinates and four controls were challenged orally with 1 ml of a 1:600 dilution of virulent FIP virus (FIP virus-DF2, passage 10, challenge titer = $10^{2.68}$ $TCID_{50}$/ml) two weeks later.

The IgG serologic response to FIP virus was determined by ELISA, Osterhaus, et al., Veterinary Quarterly 1:59(1979). The VN titers (Pedersen, et al., Am. J. Vet. Res. 44:229(1983)) were also determined.

The IgG ELISA titers of ts-FIP virus vaccinates is shown in Table 4. Pre-vaccination titers of vaccinated cats were on the day they were removed from isolation cages. Control cats had pre-vaccination ELISA titers 15 days after being removed from isolation cages. The IgG response of control cats to FIP virus is due to antibodies to cross-reacting feline enteric coronavirus (FECV) endemic in the SPF cat colony used for the experiments. As a result, the IgG response of vaccinated cats is due to FECV as well as to the vaccine virus.

Virus neutralizing titers, which in contrast to IgG ELISA titers, were neuative prior to vaccination, (Table 5) increased after each vaccination. Serum was not collected postchallenge for antibody titration. Pre exposure of vaccinated and control cats with FECV had little if any effect on the challenge since both groups had developed a cross-reactive antibody response to FIP virus. Three of four nonvaccinated control cats developed FIP and died. The fourth cat, LK2, had a clinical score of 13 (the higher the number, the more severe the symptom, with death scored as 50), three times as high as any of the nine protected vaccinates.

### Example 2: Efficacy Test 2

Twelve coronavirus negative SPF male cats (12 months of age) from Liberty Labs were used in a second vaccination-challenge test. All cats were placed in isolation cages (three cats per cage).

Six cats were vaccinated three times IN three weeks apart with ts-FIP virus. Three cats were vaccinated two times IN with ts-FIP virus and once orally. The ts-FIP virus titer was $10^{5.5}TCID_{50}$/ml. The three non-vaccinated control cats were kept in a cage separate from vaccinates.

The nine vaccinates and three controls were challenged orally with 1 ml of a 1:600 dilution of virulent FIP virus (FIP virus DF2, passage 10, challenge titer = $10^{2.15}$ $TCID_{50}$/ml ).

The serum IgG anti-Fip virus antibody response of ts-FIP virus vaccinated and non-vaccinated cats by ELISA is shown in Table 6. The greatest increase in IgG antibody titer was seen following the first and second vaccinations. No increase in titer occurred in those cats vaccinated IN a third time, confirming that one or at most two doses was sufficient to elicit protection. IgG antibody titers increased sharply following challenge. The virus neutralizing antibody titers increased following each of the three vaccinations and challenge (Table 7).

Two of three non-vaccinated control cats developed FIP and died. All of the vaccinated cats survived. Two vaccinated cats, SN1 and SY1 had temporary blood dyscrasias, primarily Doehle Bodies, low packed cell volume and an elevated body temperature. They did not, however, show any signs of icterus which is a good indicator of impending death.

Example 3: Efficacy Test 3

Eighteen coronavirus negative SPF male cats (12 months of age) from Liberty Labs were used. All cats were placed in isolation cages (two cats per cage).

Six cats were vaccinated twice IN three weeks apart with ts-FIP virus. Six cats were vaccinated once IN. The ts-FIP virus titer was $10^{5.5}$TCID$_{50}$/ml. Six additional cats were vaccinated subcutaneously (SC) with Concanavalin A (Con A) adjuvanted ts-FIP virus and kept in separate cages from the IN vaccinates. Six non-vaccinated control cats were kept in cages separate from the vaccinates.

The twelve vaccinates and six controls were challenged orally three weeks later with 1 ml of a 1:600 dilution of virulent FIP virus (FIP virus-DF2 passage 10, challenge titer = $10^{2.61}$ TCID$_{50}$/ml).

Table 8 shows the ELISA IgG titers of IN and subcutaneous ts-FIP virus vaccinated and non-vaccinated cats. Cats vaccinated twice IN and even once IN had higher mean titers than cats vaccinated SC at the time of challenge. Cats receiving two IN doses of ts-FIP virus vaccine also had higher VN titers than cats receiving a single IN dose prior to challenge (Table 9). Even one dose of ts-FIP virus administered IN stimulated higher VN titers than two doses given SC.

Peripheral blood lymphocytes from five of six cats vaccinated twice IN responded to FIP virus in the lymphocyte blastogenesis test prior to challenge. Three cats vaccinated once IN and only one cat vaccinated twice subcutaneous showed a similar response.

Two of the five, 2 dose IN vaccinates that showed a lymphocyte blastogenesis response prior to challenge plus a 2 dose IN vaccinate that failed to respond showed a strong blastogenesis response postchallenge. Only lymphocytes from a single cat vaccinated once IN and lymphocytes from no cat vaccinated twice SC responded to FIP virus following challenge.

All six cats vaccinated twice IN were solidly protected against FIP virus challenge. One of six cats (UX6) vaccinated once IN showed blood dyscrasias indicative of FIP but survived. In contrast, two of four non vaccinated cats and five of six cats vaccinated subcutaneous developed FIP and died. One of two surviving control cats (HI2) showed blood dyscrasias suggesting FIP. Most subcutaneous vaccinated cats developed FIP sooner than non-vaccinated cats.

Example 4: Preparation of Combination Vaccine

A combination vaccine consisting of ts-FIP virus combined with calicivirus and feline herpes (rhinotracheitis) virus for intranasal administration can be produced. The combination vaccine may be assembled as follows:
- 0.5 ml Feline herpes virus (TCID$_{50}$ = $10^{7.2}$ )
- 0.50 ml Calicivirus (TCID$_{50}$ = $10^{7.9}$)
- 0.5 ml ts-FIP virus (TCID$_{50}$ = $10^{5.5}$)
- 0.8 ml Stabilizer (NZ-Amine, gelatin or sucrose)

The combination vaccine can be lyophilized for shipment and storage. Prior to use the combination vaccine is rehydrated to 1.0 ml in, for example, water, saline or other diluent suitable for oral or intranasal adminstration.

Example 5: Preparation of a Subunit Vaccine

The immunogenic subunit of ts-FIP virus can be combined with whole virus or subunit components of feline distemper virus, calicivirus, feline herpes virus, feline leukemia virus and chlamydia vaccines and administered parenterally or intranasally. The combination subunit vaccine of this type may be assembled as follows:
- 0.2 ml of ts-FIP virus (TCID$_{50}$ = $10^{5.5}$)
- 0.5 ml of feline herpes virus (TCID$_{50}$ = $10^{7.2}$
- 0.25 ml of feline distemper virus (TCID$_{50}$ = $10^{6.0}$)
- 0.25 ml of calicivirus (TCID$_{50}$ = $10^{7.9}$)
- 0.5 ml of feline leukemia virus (500-3000 $\mu$g of gp 70 protein)

- 0.25 ml of chlamydia ($TCID_{50} = 10^{6.5}$)

The concentration of the various feline viruses is that amount known to elicit an effective immune response. preferably the concentrated virus is prepared in the above recommended volumes in order to have the components in a small volume suitable for administration.

Example 6: Two Component Subunit Vaccine

A two component subunit vaccine for parenteral administration can be prepared using the immunogenic subunit of ts-FIP virus combined with the immunogenic feline leukemia virus vaccine. The combination subunit vaccine may be assembled as follows:

- 0.4 ml ($TCID_{50} = 10^{5.5}$) of ts-FIP virus
- 0.4 ml (25-1000 $\mu$g) of feline leukemia virus subunit
- 0.2 ml adjuvant (aluminum hydroxide, saponin)

Example 7: Two Site Administration of Vaccines

The ts-FIP virus vaccine can be administered intranasally (0.5 ml per nostril) at the time of parenteral administration of calicivirus, feline herpes virus, feline distemper virus, feline leukemia virus and chlamydia (feline pneumonitis) combination vaccine. Concentrations of the various components would be as described in Example 4, however, the ts-FIP would be administered intranasally separate from the other virus components.

Table 4

| Serum IgG Anti FIP Virus Antibody Responses of ts-FIP Virus Vaccinated and Non-Vaccinated Cats in Efficacy Test 1 | | | | | |
|---|---|---|---|---|---|
| Cat No. | Vaccine Titer | ELISA Titer Weeks Postvaccination | | | |
| | | 0[a] | 3[b] | 6[c] | 8 |
| KU4 | $10^{5.5}$ $TCID_{50}$/ml | >1.50 [d,e] | >1.50 | 1.260 | >1.50 |
| KV4 | | .145[e] | .748 | .806 | 1.183 |
| LZ4 | | .080[e] | .590 | .719 | 1.201 |
| MJ5 | | .040[e] | .557 | .747 | 1.075 |
| MM5 | | .070[e] | .449 | .787 | 1.015 |
| Arithmetic Mean | | .367 | .769 | .864 | 1.195 |
| KT4 | $10^{3.5}$ $TCID_{50}$/ml | .074[e] | .602 | .787 | 1.420 |
| KU2 | | .129[e] | .439 | .650 | 1.179 |
| LC5 | | .095[e] | .505 | .788 | .914 |
| LR7 | | .364[e] | .698 | .676 | 1.024 |
| MG2 | | .095[e] | .676 | .845 | 1.298 |
| Arithmetic Mean | | .151 | .584 | .749 | 1.167 |
| KT3 | None | .483[f] | .881 | .823 | 1.031 |
| KV2 | | .392[f] | .715 | .827 | 1.202 |
| LD4 | | .348[f] | .702 | .842 | .973 |
| LK2 | | .499[f] | .724 | .903 | .925 |
| Arithmetic Mean | | .430 | .755 | .849 | 1.033 |

[a]First vaccination
[b]Second vaccination
[c]Third vaccination
[d]Absorbance Values measured at 405 nm. Values $\geq$.300 are positive.
[e]Serum collected on day of first vaccination
[f]Serum collected 15 days after first vaccination

Table 5

| Serum Virus Neutralization Anti-FIP Virus Antibody Titers of ts-FIP Virus Vaccinated and Non-Vaccinated Cats in Efficacy Test 1 | | | | | |
|---|---|---|---|---|---|
| Cat No. | Vaccine Titer | Reciprocal of Virus Neutralization Titer Weeks Postvaccination | | | |
| | | $0^a$ | $3^b$ | $6^c$ | 8 |
| KU4 | $10^{5.5}$ $TCID_{50}/ml$ | $<2^d$ | 64 | 192 | NT |
| KV4 | | $<2^d$ | 24 | 128 | 512 |
| LZ4 | | NT | 16 | 128 | 256 |
| MJ5 | | $<2^d$ | 192 | 192 | 384 |
| MM5 | | $<2^d$ | 16 | 48 | 512 |
| Geometric Mean | | <2 | 38 | 124 | 401 |
| KT4 | $10^{3.5}$ $TCID_{50}/ml$ | $<2^d$ | 96 | 192 | 384 |
| KU2 | | $<2^d$ | 48 | 96 | 512 |
| LC5 | | NT | 96 | 384 | NT |
| LR7 | | $<2^d$ | 64 | 96 | 192 |
| MG2 | | $<2^d$ | 96 | 256 | 768 |
| Geometric Mean | | <2 | 77 | 177 | 413 |
| KT3 | 0 | $<2^e$ | <2 | <2 | <2 |
| KV2 | | $<2^e$ | <2 | <2 | <2 |
| LD4 | | $<2^e$ | <2 | <2 | <2 |
| LK2 | | $<2^e$ | <2 | <2 | <2 |
| Geometric Mean | | <2 | <2 | <2 | <2 |

[a]First vaccination
[b]Second vaccination
[c]Third vaccination
[d]Serum collected on day of first vaccination
[e]Serum collected 15 days after first vaccination

Table 6

| Serum IgG Anti FIP Virus Antibody Response of ts-FIP Virus Vaccinated and Non-Vaccinated Cats in Efficacy Test 2 | | | | | | | |
|---|---|---|---|---|---|---|---|
| Cat No. | No of Times Vaccinated IN | ELISA Titer Weeks Postvaccination | | | | | |
| | | -1[a] | 3[b] | 3[c] | 3[d] | 12 | 18 |
| SD1 | 3 | .110[e] | .348 | .683 | .633 | 1.078 | 1.249 |
| SL2 | | .135 | .346 | .669 | .482 | 1.089 | 1.264 |
| SN1 | | .086 | .483 | .812 | .776 | 1.094 | 1.397 |
| SO2 | | .196 | .321 | .573 | .620 | 1.095 | 1.094 |
| ST2 | | .164 | .268 | .609 | .532 | 1.275 | 1.229 |
| SY3 | | .147 | .532 | 1.013 | .768 | 1.232 | 1.424 |
| Arithmetic Mean | | .140 | .383 | .726 | .635 | 1.144 | 1.273 |
| RY5 | 2[f] | .106 | .383 | .740 | .725 | .987 | 1.003 |
| SI2 | | .282 | .299 | .718 | .738 | 1.130 | 1.054 |
| SY1 | | .106 | .383 | .868 | .792 | 1.271 | 1.421 |
| Arithmetic Mean | | .165 | .355 | .775 | .752 | 1.129 | 1.162 |
| RZ3 | 0 | .123 | .052 | .063 | .073 | .589 | Dead |
| SH4 | | .074 | .030 | .047 | .049 | .417 | Dead |
| SL5 | | .155 | .051 | .074 | .040 | .291 | 0.609 |
| Arithmetic Mean | | .117 | .044 | .061 | .054 | .432 | 0.609 |

[a]First vaccination

[b]Second vaccination

[c]Third vaccination

[d]Challenged 4 weeks post third vaccination

[e]Absorbance Values measured at 405 nm. Values ≥.300 are positive.

[f]Vaccinated a third time orally.

Table 7

| Serum Virus Neutralization Anti-FIP Virus Antibody Titers of ts-FIP Virus Vaccinated and Non-Vaccinated Cats in Efficacy Test 2 | | | | | | | |
|---|---|---|---|---|---|---|---|
| Cat No. | No of Times Vaccinated IN | Reciprocal of Virus Neutralization Titer Weeks Postvaccination | | | | | |
| | | -1[a] | 3[b] | 6[c] | 9[d] | 12 | 18 |
| SD1 | 1 | 0 | 64 | 384 | NT | 192 | 96 |
| SL2 | | 0 | 96 | 1024 | 240 | 7200 | 9600 |
| SN1 | | 0 | 48 | 256 | 360 | 4800 | 9600 |
| SO2 | | 0 | 48 | 192 | NT | 900 | 192 |
| ST2 | | 0 | 192 | 768 | 120 | NT | 64 |
| SY3 | | 0 | 48 | 768 | 360 | 7200 | 7200 |
| Geometric Mean | | 0 | 71 | 474 | 247 | 2122 | 960 |
| RY5 | 2[e] | 0 | 48 | 192 | NT | 192 | 600 |
| SI2 | | 0 | 48 | 256 | 120 | 128 | 64 |
| SY1 | | 0 | 96 | 1024 | 360 | 900 | 9600 |
| Geometric Mean | | 0 | 60 | 369 | 208 | 281 | 717 |
| RZ3 | 0 | 0 | 0 | 8 | 0 | 900 | Dead |
| SH4 | | 0 | 0 | 1 | 0 | 600 | Dead |
| SL5 | | 0 | 0 | 1 | 0 | 2 | 3 |
| Geometric Mean | | 0 | 0 | 2 | 0 | 81 | 3 |

[a]First vaccination
[b]Second vaccination
[c]Third vaccination
[d]Challenged 4 weeks post third vaccination
[e]Vaccinated a third time orally.

Table 8

| Serum IgG Anti FIP Virus Antibody Response of Intranasal and Subcutaneous ts-FIP Virus Vaccinated and Non-Vaccinated Cats in Efficacy Test 3 | | | | | | | |
|---|---|---|---|---|---|---|---|
| Cat No. | Route/No. of Vaccinations | ELISA Titer Weeks Postvaccination | | | | | |
| | | $0^a$ | $3^b$ | $8^c$ | 11 | 14 | 16 |
| SU4 | IN/1 | $0.028^d$ | 0.012 | 0.399 | 0.476 | 0.334 | 0.474 |
| TR3 | | 0.017 | 0.007 | 0.603 | 0.552 | 0.428 | 0.697 |
| TT2 | | 0.018 | 0.011 | 0.592 | 1.153 | 0.745 | 1.321 |
| UN3 | | 0.012 | 0.010 | 0.674 | 0.948 | 0.673 | 1.400 |
| UO4 | | 0.064 | 0.021 | 0.329 | 0.414 | 0.397 | 0.269 |
| UX6 | | 0.035 | 0.020 | 0.683 | 1.116 | 0.856 | 1.475 |
| Arith. Mean | | 0.029 | 0.014 | 0.547 | 0.776 | 0.574 | 0.939 |
| OH2 | IN/2 | 0.016 | 0.351 | 0.858 | 0.537 | 0.443 | 0.878 |
| PQ4 | | 0.104 | 0.095 | 0.578 | 0.682 | 0.610 | 0.704 |
| QK4 | | 0.013 | 0.191 | 0.660 | 0.502 | 0.498 | 0.727 |
| UE1 | | 0.029 | 0.223 | 0.881 | 1.193 | 0.885 | 1.543 |
| US1 | | 0.018 | 0.151 | 0.796 | 1.212 | 0.934 | 1.629 |
| US2 | | 0.016 | 0.176 | 0.773 | 0.731 | 0.514 | 0.886 |
| Arith. Mean | | 0.033 | 0.198 | 0.758 | 0.809 | 0.647 | 1.061 |
| QG2 | SC/2 | 0.030 | 0.021 | 0.229 | Dead | Dead | Dead |
| QJ1 | | 0.125 | 0.142 | 0.235 | 0.828 | Dead | Dead |
| QY3 | | 0.189 | 0.108 | 0.603 | 1.034 | Dead | Dead |
| RG3 | | 0.017 | 0.150 | 0.516 | 0.981 | 1.37 | Dead |
| TU4 | | 0.031 | 0.042 | 0.235 | 0.495 | 0.135 | 0.121 |
| UG1 | | 0.018 | 0.243 | 0.507 | 1.388 | Dead | Dead |
| Arith. Mean | | 0.068 | 0.118 | 0.388 | 0.945 | 0.752 | 0.121 |
| HH5 | Control | $NT^e$ | 0.041 | 0.042 | 0.403 | Dead | Dead |
| HJ4 | | NT | 0.015 | 0.062 | 0.561 | NT | 1.205 |
| HG2 | | NT | 0.030 | 0.059 | 0.744 | 0.743 | Dead |
| HI2 | | NT | 0.007 | 0.048 | 0.298 | 0.514 | 1.225 |
| Arith. Mean | | | 0.023 | 0.053 | 0.501 | 0.629 | 1.215 |

[a]First Vaccination of cats receiving 2 doses

[b]Second vaccination of cats receiving 2 doses and first vaccination of cats receiving one dose

[c]Titers one week before challenge

[d]Absorbance values measured at 405 nm. Values ≥0.300 are positive.

[e]Not Tested.

13

Table 9

| Serum Virus Neutralization Anti-FIP Virus Antibody Titers of ts-FIP Virus Intranasal and Subcutaneous Vaccinated and Nonvaccinated Cats in Efficacy Test 3 | | | | | | | |
|---|---|---|---|---|---|---|---|
| Cat No. | Route/No. of Vaccinations | Reciprocal of Virus Neutralization Titer Weeks Postvaccination | | | | | |
| | | $0^a$ | $3^b$ | $8^c$ | 11 | 14 | 16 |
| SU4 | IN/1 | <4 | <8 | 64 | 30 | 20 | 20 |
| TR3 | | <2 | <8 | 256 | 120 | 120 | 40 |
| TT2 | | <2 | <8 | 96 | 1920 | 640 | 480 |
| UN3 | | <2 | <8 | 512 | 320 | 3840 | 1280 |
| UO4 | | 3 | 8 | 48 | 40 | 30 | 20 |
| UX6 | | 4 | 8 | 192 | 960 | 960 | 1920 |
| Geometric Mean | | <2 | <8 | 140 | 210 | 235 | 163 |
| OH2 | IN/2 | <2 | 48 | 256 | 120 | 60 | 80 |
| PQ4 | | <2 | 96 | 512 | 120 | 60 | 60 |
| QK4 | | <2 | 96 | 192 | 80 | 40 | 120 |
| UE1 | | <2 | 128 | 768 | 1920 | 2560 | 2560 |
| US1 | | <2 | 96 | 512 | 160 | 640 | 960 |
| US2 | | <2 | 64 | 512 | 120 | 240 | 80 |
| Geometric Mean | | <2 | 84 | 414 | 187 | 196 | 220 |
| QG2 | SC | <2 | <8 | 8 | Dead | Dead | Dead |
| QJ1 | | <2 | <8 | <8 | 1280 | Dead | Dead |
| QY3 | | <2 | <8 | 128 | 640 | Dead | Dead |
| RG3 | | 24 | 8 | 64 | 1920 | Dead | Dead |
| TU4 | | <2 | <8 | 12 | <20 | 20 | <20 |
| UG1 | | <2 | 12 | 32 | 640 | Dead | Dead |
| Geometric Mean | | <2 | <8 | 24 | 399 | 20 | <20 |
| HH5 | Control | $NT^d$ | NT | <2 | 120 | Dead | Dead |
| HJ4 | | NT | NT | <2 | 120 | 480 | 480 |
| HG2 | | NT | NT | <2 | 960 | Dead | Dead |
| HI2 | | NT | NT | <2 | 240 | 3480 | 640 |
| Geometric Mean | | | | <2 | 240 | 1292 | 554 |

[a]First Vaccination of cats receiving 2 doses
[b]Second vaccination of cats receiving 2 doses and first vaccination of cats receiving one dose
[c]Titers one week before challenge
[d]Not Tested.

The above description and examples fully disclose the invention including preferred embodiments thereof.

**Claims**
**Claims for the following Contracting States : AT, BE, CH, DE, FR, GB, IT, LI, LU, NL, SE**

1. A feline infectious peritonitis (FIP) vaccine for oral or intranasal administration which comprises an effective amount of an attenuated temperature sensitive (ts) FIP virus.

2. The vaccine of claim 1 wherein the FIP virus is attenuated by the serial passage of virulent FIP virus isolated from the tissue or organs of infected felines by passaging the virus in susceptible cells, the number of passages being at least about 60.

3. The vaccine of claim 2 wherein the temperature sensitive FIP virus is attenuated by passage in feline kidney cells at between 30°C and 35°C at least about 60 times.

4. The vaccine of claim 2 or 3 wherein the attenuated FIP virus is made temperature sensitive by exposure of the FIP virus to a mutagenic agent.

5. The vaccine of claim 4 wherein the mutagenic agent is ultraviolet light.

6. The vaccine of claim 5 wherein the mutagenesis is performed by exposing the FIP virus to ultraviolet light for 3 to 5 minutes.

7. The vaccine of claim 4 wherein the attenuated temperature sensitive FIP virus shows a difference in growth of about $1 \times 10^2$ $TCID_{50}$ between 31°C and 39°C.

8. The vaccine of claim 4 which contains $10^2$ to $10^7$ $TCID_{50}$ of temperature sensitive FIP virus per ml.

9. The vaccine of claim 8 which contains $10^{5.5}$ $TCID_{50}$ of ts-FIP virus per ml when cultured at 31°C.

10. A method of preparation of a vaccine against FIP virus comprising :
    1) attenuating FIP virus by high passage in susceptible cells;
    2) exposing the attenuated FIP virus to a mutagenic agent;
    3) culturing attenuated FIP virus from step (2) which is temperature sensitive; and
    4) collecting the attenuated ts-FIP virus.

11. An attenuated, temperature sensitive FIP virus for use in therapy.

12. FIP virus which is useful in the production of a vaccine wherein the FIP virus is attenuated and temperature sensitive.

13. The FIP virus of claim 12 which exhibits growth at 39°C which is at least $1 \times 10^2$ $TCID_{50}$ lower than growth at 31°C.

14. The FIP virus of claim 12 wherein the FIP virus is attenuated by multiple passages in culture with feline kidney cells.

15. The FIP virus of claim 12 wherein the FIP virus is made temperature sensitive by exposure to a mutagenic agent.

16. The FIP virus of claim 12 wherein the FIP virus is effective at inducing immunity in felines to infection by FIP at a dosage of from $10^2$ to $10^7$ $TCID_{50}$.

17. A vaccine dosage unit for inducing immunity to infection by FIP virus which comprises 0.5 to 1.5 ml of a liquid, suitable for intranasal or oral administration to a feline, containing $10^2$ to $10^7$ $TCID_{50}$ of an attenuated temperature sensitive FIP virus.

18. A combination vaccine for oral or intranasal administration capable of inducing immunity in felines to infection by FIP virus and one or more other pathogenic organisms or viruses comprising attenuated ts-FIP virus and one or more other antigens protective against infection by another pathogenic organism or virus.

19. A combination vaccine according to claim 18 which comprises attenuated ts-FIP virus, Feline herpes virus and calici virus.

**Claims for the following Contracting States : ES, GR**

1. A method of preparation of a vaccine against FIP virus comprising :
    1) attenuating FIP virus by high passage in susceptible cells;
    2) exposing the attenuated FIP virus to a mutagenic agent;

3) culturing attenuated FIP virus from step (2) which is temperature sensitive; and

4) collecting the attenuated ts-FIP virus.

2. The method according to claim 1 wherein the FIP virus is isolated from the tissue or organs of a feline infected with virulent FIP prior to attenuation.

3. The method according to claim 1 wherein the FIP virus is attenuated by successive passages, at least about 60, in feline kidney cells.

4. The method according to claim 1 wherein the FIP virus is made temperature sensitive by exposing the high passage FIP virus to a mutagenic agent.

5. The method according to claim 1 wherein the FIP virus is made temperature sensitive by exposing the high passage FIP virus to ultraviolet light for 3 to 5 minutes.

6. An attenuated, temperature sensitive FIP virus for use in therapy.

7. An attenuated temperature sensitive FIP virus for use in a vaccine.

8. FIP virus which is useful in the production of a vaccine wherein the FIP virus is attenuated and temperature sensitive.

9. The FIP virus of claim 8 which exhibits growth at 39°C which is at least $1 \times 10^2$ $TCID_{50}$ lower than growth at 31°C.

10. The FIP virus of claim 8 wherein the FIP virus is attenuated by multiple passages in culture with feline kidney cells.

11. The FIP virus of claim 8 wherein the FIP virus is made temperature sensitive by exposure to a mutagenic agent.

12. The FIP virus of claim 8 wherein the FIP virus is effective at inducing immunity in felines to infection by FIP at a dosage of from $10^2$ to $10^7$ $TCID_{50}$.

13. A method according to any one of claims 1 to 5 which comprises the further step of incorporating into the vaccine an effective amount of one or more other antigens protective against infection by another pathogenic organism or virus.

14. A method according to claim 13 in which the vaccine prepared comprises attenuated ts-FIP virus, Feline herpes virus and calici virus.

**Patentansprüche**

**Patentansprüche für folgende Vertragsstaaten : AT, BE, CH, DE, FR, GB, IT, LI, LU, NL, SE**

1. Feliner infektiöser Peritonitis (FIP)-Impfstoff zur oralen oder intranasalen Applikation, enthaltend eine wirksame Menge eines attenuierten temperaturempfindlichen (ts) FIP-Virus.

2. Impfstoff nach Anspruch 1, wobei das FIP-Virus durch Reihenpassage von virulentem FIP-Virus, isoliert aus dem Gewebe oder Organen von infizierten Felinen durch Passagieren des Virus in empfänglichen Zellen attenuiert worden ist, und die Zahl der Passagen mindestens etwa 60 beträgt.

3. Impfstoff nach Anspruch 2, wobei das temperaturempfindliche FIP-Virus attenuiert wird durch mindestens 60 Passagen in felinen Nierenzellen bei Temperaturen von 30 bis 35°C.

4. Impfstoff nach Anspruch 2 oder 3, wobei das attenuierte FIP-Virus durch Behandeln des FIP-Virus mit einem mutagenen Mittel temperaturempfindlich gemacht wird.

5. Impfstoff nach Anspruch 4, wobei das mutagene Mittel ultraviolettes Licht ist.

16

6. Impfstoff nach Anspruch 5, wobei die Mutagenese durch 3 bis 5minütige Belichtung des FIP-Virus mit ultraviolettem Licht durchgeführt wird.

7. Impfstoff nach Anspruch 4, wobei das attenuierte, temperaturempfindliche FIP-Virus einen Unterschied im Wachstum von etwa 1 x $10^2$ $TCID_{50}$ zwischen 31°C und 39°C zeigt.

8. Impfstoff nach Anspruch 4, enthaltend $10^2$ bis $10^7$ $TCID_{50}$ des temperaturempfindlichen FIP-Virus pro Milliliter.

9. Impfstoff nach Anspruch 8, enthaltend $10^{5,5}$ $TCID_{50}$ des ts-FIP-Virus pro Milliliter bei 31°C kultiviert.

10. Verfahren zur Herstellung eines Impfstoffes sagen FIP-Virus, umfassend:
    1) Attenuieren des FIP-Virus durch hohe Passage in empfänglichen Zellen;
    2) Behandlung des attenuierten FIP-Virus mit einem mutagenen Mittel;
    3) Kultivieren des in Stufe (2) erhaltenen temperaturempfindlichen attenuierten FIP-Virus und
    4) Sammeln des attenuierten ts-FIP-Virus.

11. Ein attenuierter, temperaturempfindlicher FIP-Virus zur Verwendung in der Therapie.

12. FIP-Virus, geeignet zur Produktion eines Impfstoffes, in dem das FIP-Virus attenuiert und temperaturempfindlich ist.

13. FIP-Virus nach Anspruch 12, das bei 39°C ein Wachstum aufweist, das um um 1 x $10^2$ $TCID_{50}$ niedriger ist als das Wachstum bei 31°C.

14. FIP-Virus nach Anspruch 12, wobei das FIP-Virus durch mehrfache Passagen in einer Kultur mit felinen Nierenzellen attenuiert ist.

15. FIP-Virus nach Anspruch 12, wobei das FIP-Virus durch Behandlung mit einem mutagenen Mittel temperaturempfindlich gemacht ist.

16. FIP-Virus nach Anspruch 12, wobei das FIP-Virus wirksam ist zur Induktion von Immunität in Felinen gegenüber Infektion durch FIP bei einer Dosis von $10^2$ bis $10^7$ $TCID_{50}$.

17. Impfstoff-Dosiseinheit zur Induktion von Immunität gegenüber Infektion durch FIP-Virus, enthaltend 0,5 bis 1,5 ml einer zur intranasalen oder oralen Applikation an Feline geeigneten Flüssigkeit, die $10^2$ bis $10^7$ $TCID_{50}$ eines attenuierten und temperaturempfindlichen FIP-Virus enthält.

18. Ein Kombinations-Impfstoff zur oralen oder intranasalen Applikation, der in der Lage ist Immunität in Felinen gegenüber Infektion durch FIP-Virus und einem oder mehreren anderen pathogenen Organismen oder Viren zu erzeugen, enthaltend ein attenuiertes ts-FIP-Virus und eines oder mehrere andere Antigene, die Schutz gegen Infektion durch andere pathogene Organismen oder Viren verleihen.

19. Ein Kombinations-Impfstoff nach Anspruch 18, enthaltend ein attenuiertes ts-FIP-Virus, felines Herpes-Virus und Calici-Virus.

**Patentansprüche für folgende Vertragsstaaten : ES, GR**

1. Verfahren zur Herstellung eines Impfstoffes gegen FIP-Virus, umfassend:
    1) Attenuieren des FIP-Virus durch hohe Passage in empfänglichen Zellen;
    2) Behandlung des attenuierten FIP-Virus mit einem mutagenen Mittel;
    3) Kultivieren des in Stufe (2) erhaltenen temperaturempfindlichen attenuierten FIP-Virus und
    4) Sammeln des attenuierten ts-FIP-Virus.

2. Verfahren nach Anspruch 1, wobei das FIP-Virus aus dem Gewebe oder Organen einer Felinen isoliert worden ist, die mit virulentem FIP-Virus vor der Attenuierung infiziert wurde.

**3.** Verfahren nach Anspruch 1, wobei das FIP-Virus durch aufeinanderfolgende mindestens 60malige Passagen in felinen Nierenzellen attenuiert wird.

**4.** Verfahren nach Anspruch 1, wobei das FIP-Virus durch Behandlung des hohen Passagen unterworfenen FIP-Virus mit einem mutagenen Mittel temperaturempfindlich gemacht wird.

**5.** Verfahren nach Anspruch 1, wobei das FIP-Virus durch 3 bis 5minütiges Bestrahlen des hohen Passagen unterworfenen FIP-Virus mit ultraviolettem Licht temperaturempfindlich gemacht wird.

**6.** Ein attenuiertes, temperaturempfindliches FIP-Virus zur Verwendung in der Therapie.

**7.** Ein attenuiertes, temperaturempfindliches FIP-Virus zur Verwendung in einem Impfstoff.

**8.** FIP-Virus, geeignet zur Herstellung eines Impfstoffes, wobei das FIP-Virus attenuiert und temperaturempfindlich ist.

**9.** FIP-Virus nach Anspruch 8, das bei 39°C ein Wachstum aufweist, das um um $1 \times 10^2$ TCID$_{50}$ niedriger ist als das Wachstum bei 31°C.

**10.** FIP-Virus nach Anspruch 8, wobei das FIP-Virus durch mehrfache Passagen in Kulturen mit felinen Nierenzellen attenuiert ist.

**11.** FIP-Virus nach Anspruch 8, wobei das FIP-Virus durch Behandlung mit einem mutagenen Mittel temperaturempfindlich ist.

**12.** FIP-Virus nach Anspruch 8, wobei das FIP-Virus wirksam ist zur Induktion von Immunität in Felinen gegen Infektionen durch FIP bei einer Dosis von $10^2$ bis $10^7$ TCID$_{50}$.

**13.** Verfahren nach einem der Ansprüche 1 bis 5, dadurch gekennzeichnet, daß man dem Impfstoff eine wirkungsvolle Menge eines oder mehrerer Antigene einverleibt, die Schutz gegen andere pathogene Organismen oder Viren verleihen.

**14.** Verfahren nach Anspruch 13, wobei der hergestellte Impfstoff attenuiertes ts-FIP-Virus, felines Herpes-Virus und Calici-Virus enthält.

**Revendications**
**Revendications pour les Etats contractants suivants : AT, BE, CH, DE, FR, GB, IT, LI, LU, NL, SE**

**1.** Vaccin contre la péritonite infectieuse féline (FIP) pour l'administration orale ou intranasale, qui comprend une quantité efficace d'un virus FIP atténué sensible à la température (st).

**2.** Vaccin suivant la revendication 1, dans lequel le virus FIP est atténué par le passage on série du virus FIP virulent isolé à partir de tissu ou d'organes de félins infectés par passage du virus dans des cellules sensibles, le nombre de passages étant d'au moins environ 60.

**3.** Vaccin suivant la revendication 2, dans lequel le virus FIP sensible à la température est atténué par passage dans des cellules rénales félines à une température comprise entre 30°C et 35°C, le nombre de passages étant d'au moins environ 60.

**4.** Vasein suivant les revendications 2 ou 3, dans lequel le virus FIP atténué est rendu sensible à la température par exposition du virus FIP à un agent mutagène.

**5.** Vaccin suivant la revendication 4, dans lequel l'agent mutagène est une lumière ultra-violette.

**6.** Vaccin suivant la revendication 5, dans lequel la mutagenèse est réalisée par exposition du virus FIP à une lumière ultra-violette pondant 3 à 5 minutes.

18

**7.** Vaccin suivant la revendication 4, dans lequel le virus FIP atténué sensible à la température présente une différence de croissance d'environ $1 \times 10^2$ $TCID_{50}$ entre 31°C et 39°C.

**8.** Vaccin suivant la revendication 4, qui contient de $10^2$ à $10^7$ $TCID_{50}$ de virus FIP sensible à la température par ml.

**9.** Vaccin suivant la revendication 8, qui contient $10^{5,5}$ $TCID_{50}$ de virus FIP sensible à la température par ml, lorsqu'il est cultivé à 31°C.

**10.** Procédé de préparation d'un vaccin contre le virus FIP, comprenant :
(1) l'atténuation du virus FIP par passage élevé dans des cellules sensibles;
(2) l'exposition du virus FIP atténué à un agent mutagène;
(3) la culture du virus FIP atténué provenant de l'étape (2) qui est sensible à la température, et
(4) la collection du virus FIP atténué sensible à la température.

**11.** Virus FIP atténué, sensible à la température utilisable en thérapie.

**12.** Virus FIP, qui est utile dans la production d'un vaccin dans lequel le virus FIP est atténué et sensible à la température.

**13.** Virus FIP suivant la revendication 12, qui présente une croissance à 39°C qui est d'au moins $1 \times 10^2$ $TCID_{50}$ inférieure à la croissance à 31°C.

**14.** Virus FIP suivant la revendication 12, dans lequel le virus FIP est atténué par de multiples passages en culture avec des cellules rénales félines.

**15.** Virus FIP suivant la revendication 12, dans lequel le virus FIP est rendu sensible à la température par exposition à un agent mutagène.

**16.** Virus FIP suivant la revendication 12, dans lequel le virus FIP est efficace pour induire une immunité chez des félins contre une infection par FIP à une dose de $10^2$ à $10^7$ $TCID_{50}$.

**17.** Dose unitaire de vaccin pour induire une immunité contre une infection par FIP, qui comprend 0,5 à 1,5 ml d'un liquide, convenable pour l'administration intranasale ou orale à un félin, contenant de $10^2$ à $10^7$ $TCID_{50}$ d'un virus FIP atténué sensible à la température.

**18.** Vaccin associé pour l'administration orale ou intranasale, capable d'induire une immunité chez les félins contre une infection par le virus FIP et un ou plusieurs autres organismes ou virus pathogènes, comprenant un virus FIP atténué sensible à la température et un ou plusieurs autres antigènes protégeant contre une infection par un autre organisme ou virus pathogène.

**19.** Vaccin associé suivant la revendication 18, qui comprend un virus FIP atténué sensible à la température, un virus de l'herpès félin et un virus calici.

**Revendications pour les Etats contractants suivants : ES, GR**

**1.** Procédé de préparation d'un vaccin contre le virus FIP, comprenant :
(1) l'atténuation du virus FIP par passage élevé dans des cellules sensibles;
(2) l'exposition du virus FIP atténué à un agent mutagène;
(3) la culture du virus FIP atténué provenant de l'étape (2) qui est sensible à la température, et
(4) la collection du virus FIP atténué sensible à la température.

**2.** Procédé suivant la revendication 1, dans lequel le virus FIP est isolé à partir de tissu ou d'organes d'un félin infecté avec du FIP virulent avant atténuation.

**3.** Procédé suivant la revendication 1, dans lequel le virus FIP est atténué par des passages successifs dans des cellules rénales félines, le nombre de passages étant d'au moins environ 60.

**4.** Procédé suivant la revendication 1, dans lequel le virus FIP est rendu sensible à la température par exposition du virus FIP obtenu après un passage élevé à un agent mutagène.

**5.** Procédé suivant la revendication 1, dans lequel le virus FIP est rendu sensible à la température par exposition du virus FIP obtenu après un passage élevé à uns lumière ultra-viollete pendant 3 à 5 minutes.

**6.** Virus FIP atténué, sensible à la température utilisable en thérapie.

**7.** Virus FIP atténué et sensible à la température utilisable dans un vaccin.

**8.** Virus FIP qui est utile dans la production d'un vaccin dans lequel le virus FIP est atténué et sensible à la température

**9.** Virus FIP suivant la revendication 8, qui présente une croissance à $39°C$ qui est d'au moins $1 \times 10^2$ $TCID_{50}$ inférieure à là croissance à $31°C$.

**10.** Virus FIP suivant la revendication 8, dans lequel le virus FIP est atténué par de multiples passages en culture avec des cellules rénales félines.

**11.** Virus FIP suivant la revendication 8, dans lequel le virus FIP est rendu sensible à la température par exposition à un agent mutagène.

**12.** Virus FIP suivant la revendication 8, dans lequel le virus FIP est efficace pour induire une immunité chez des félins contre une infection par FIP à une dose de $10^2$ à $10^7$ $TCID_{50}$.

**13.** Procédé suivant l'une quelconque des revendications 1 à 5, qui comprend l'étape supplémentaire d'incorporation dans le vaccin d'une quantité efficace d'un ou de plusieurs autres antigènes protégeant contre une infection par un autre organisme ou virus pathogène.

**14.** Procédé suivant la revendication 13, dans lequel le vaccin préparé comprend un virus FIP atténué sensible à la température, un virus de l'herpès félin et un virus calici.